# EUROPEAN PATENT SPECIFICATION

(11) **EP 3 801 887 B1**
(45) Date of publication and mention of the grant of the patent: **29.06.2022**
(21) Application number: 19745278.2
(22) Date of filing: 04.06.2019
(51) Int. Cl.: B01J 23/78, B01J 23/889, B01J 37/06, B01J 37/03, B01J 37/08, B01J 35/00, C10G 2/00, C07C 1/04

(54) **BULK-METAL CRYSTALLINE TRANSITION METAL BASED HETEROGENEOUS CATALYSTS, METHODS OF MAKING AND USES THEREOF**
HETEROGENE KATALYSATOREN AUF BASIS VON MASSEMETALL UND KRISTALLINEM ÜBERGANGSMETALL, VERFAHREN ZUR HERSTELLUNG UND VERWENDUNGEN DAVON
CATALYSEURS HÉTÉROGÈNES À BASE DE MÉTAUX DE TRANSITION CRISTALLINS DE MÉTAL MASSIFS, LEURS PROCÉDÉS DE FABRICATION ET LEURS UTILISATIONS

(30) Priority: 05.06.2018 US 201862680725 P
(43) Date of publication of application: 14.04.2021
(73) Proprietor: SABIC Global Technologies B.V., 4612 PX Bergen op Zoom (NL)
(72) Inventor: HAIDER, Muhammad H., Riyadh, 11551 (SA); AL-HARBI, Yasser T., Riyadh, 11551 (SA); AL-ZENAIDI, Ahmed S., Riyadh, 11551 (SA)
(74) Representative: Patentanwälte Bressel und Partner mbB
(86) International application number: PCT/IB2019/054642
(87) International publication number: WO 2019/234631

(56) References cited:
- WO-A1-2018/040798
- WO-A1-2018/109718
- WO-A2-2011/027921
- WO-A2-2015/015313
- US-A1- 2009 111 684

## Description

### BACKGROUND OF THE INVENTION

### A. Field of the Invention

The invention generally concerns bulk-metal crystalline catalysts that can include a first transition metal core surrounded by a silica-alkaline earth metal framework crystal lattice and includes at least one transition metal atoms bound to periphery of the framework crystal lattice. The two transition metals can be iron (Fe), cobalt (Co), manganese (Mn), rhodium (Rh), ruthenium (Ru) and combinations thereof.

### B. Description of Related Art

Conventional iron (Fe) and cobalt (Co) based catalysts can have advantages over other transition metal *(e.g.,* nickel, copper, cerium, rhodium, ruthenium, *etc.*) based catalysts for conversion of synthesis gas ("syngas") to olefins *via* a Fischer Tropsch process. Synthesis gas is a mixture of hydrogen (H₂) and carbon monoxide (C), and optional carbon dioxide (CO₂). In this process, carbon monoxide and hydrogen in synthesis gas react over a metal-based catalyst to form olefins as shown in the following reactions:

CO + M → M-CO (CO adsorption); (1)

M-CO→M-C + M-O (CO dissociation); (2)

M-C + [H] → M-CH + [H] → M-CH₂; (H₂ adsorption/methylene formation) (3)

M-CH₂ + [H] → M-CH₃ → M······CH₂-CH₃ (Chain propagation/termination in H₂) (4)

where M is a metal atom of the catalyst, [H] is hydrogen atom of the hydrogen gas in synthesis gas, and CH is an intermediate for a methylene group. The advantages of using a Fischer-Tropsch reaction for production of olefins has been attributed to the easy dissociative adsorption of CO, reasonable hydrogen adsorption, easy reducibility of metal oxide surface species and economically feasible (availability and cost). Further advantages include cobalt being stable for long durations and iron being very active and having high water-gas shift (WGS) activity, which can be required for low hydrogen based feedstocks. However, both catalyst have disadvantages. For example, cobalt based catalysts can have low WGS activity and can produce long straight chain products whereas iron based catalysts can have higher WGS activity and produce short chain products. However iron based catalysts can have a short lifetime due to its strong ability to form carbon deposits leading to deactivation.

To address these disadvantages of Fischer-Tropsch catalysts, promotion of Co with manganese (Mn) and/or Fe have been described. Promotion with Mg can shift the reaction towards a more olefinic product distribution as compared to unpromoted cobalt, while addition of iron can promote higher WGS activity. By way of example, U.S. Patent No. 9,545,620 to Karim et al. describes a catalyst that includes Co, Mg, Fe, and hydrophilic silica as a binder for the production of olefins from syngas. While this catalyst had high conversion of syngas, it produced undesirable quantities of methane (*e.g.,* greater than 30 mol.%). In another example, Sainna et al. (Journal of Thermodynamics & Catalysis, 2016, 7, 172) describes a mesoporous silica support impregnated with Co, Fe, and Mg.

WO 2015/015313 A2 discloses a Fischer-Tropsch catalyst sytem having the composition CoMnNiMgCuSi made of the mixture of two catalyst precursors each one containing their own lattice, namely CoMnSi and NiCuMgSi.

While various supported Co, Fe, Mg promoted catalysts are known, these catalyst suffer in high selectivity to methane and/or involve complicated methodology to prepare.

### SUMMARY OF THE INVENTION

A solution to some of the problems discussed above concerning Fischer-Tropsch conversion of syngas to olefins has been discovered. The solution is premised on a bulk metal crystalline catalyst that is a bimetallic catalyst or a multimetallic (*e.g.,* 3 or more catalytic metals) catalyst. The catalyst can include a first catalytic active transition metal core *(e.g.,* Fe) surrounded by a silica-alkaline earth metal framework crystal lattice that can include at least one additional catalytic active transition metals (*e.g.,* Co, manganese (Mn), rhodium (Rh), ruthenium (Ru) or combinations thereof) bound to the periphery of the framework crystal lattice. A non-limiting example of a bimetallic bulk metal crystalline catalyst would be a CoFeSiMgO type catalyst. A non-limiting example of a trimetallic bulk metal crystalline catalyst would be a MnCoFeSiMgO type catalyst. Notably, and as exemplified in non-limiting Examples, the catalyst can be stable for very long time duration *(e.g.,* 400 hours or more) and/or can produce short chain products *(e.g.,* C2-C4 olefinic products) along with high WGS activity. Without wishing to be bound by theory, it is believed that putting the Fe metal in the core of the crystal lattice inhibits deactivation of the catalyst.

In one aspect of the current invention, bulk metal crystalline catalysts are described. The catalyst includes at least two transitions metals and has a formula of (M¹)*ₐ*(M²)*_{b}*(Fe)*_{c}*Si*ₓ*M³_{y}O*_{z}* where M¹ and M² are the at least two transition metals selected from the group consisting of cobalt, ruthenium, rhodium manganese, and combinations thereof M³ is an alkaline earth metal, and 0.01 ≤ *a* < 1, 0.07 ≤ *b* <1, 0.01 ≤ *c* < 1, 0.03 ≤ *x* < 1, 0.26 < *y* ≤ 1, and z is balances the valence of the catalyst. In some embodiments, z is 0.30 to 0.40, or 0.35 to 0.36. The alkaline earth metal (M³) can include magnesium, calcium, strontium, barium or oxides and mixtures thereof, preferably magnesium. In some embodiments, the first transition metal core is Fe metal surrounded by a silica-magnesia framework crystal lattice and includes cobalt (Co) and manganese (Mn) atoms bound to periphery of the framework crystal lattice. Such a catalyst can have a formula of (Mn)*ₐ*(Co)*_{b}*Fe*_{c}*Si*ₓ*(Mg)*_{y}*O*_{z}* where 0.01 ≤ *a <* 1, 0.07 ≤ *b* <1, 0.01 ≤ *c* < 1, 0.03 ≤ *x* < 1, 0.26 < *y* ≤ 1, and z is 0.3 to 0.4, preferably 0.35. In some instances, the catalyst is absent, or substantially absent (*i.e.,* less than 1 wt.%, or 0 wt.%) a binder.

In another aspect of the invention, methods for preparing the catalyst of the present invention are described. A method can include the steps of: (a) obtaining a solution of a silicon precursor material (e.g., tetra-alkyl silicate such as tetraethyl orthosilicate (TEOS), an alkaline earth metal precursor material (*e.g.,* magnesium chloride) and at least one transition metal precursor materials *(e.g.,* iron citrate, cobalt nitrate, magnesium nitrate, *etc*.); (b) precipitating a silica/alkaline-earth metal/transition metals agglomerate from the solution, the silica/alkaline-earth metal/transition metals agglomerate can include the first transition metal core bound to a silica-alkaline earth metal framework crystal lattice and the second transition metal on the periphery of the framework crystal lattice and precursor material; and (c) contacting the precipitated material with an oxidizing agent (*e.g.*, hydrogen peroxide (H₂O₂) to remove the precursor material and produce a bulk metal crystalline catalyst having a silica-alkaline earth metal crystal lattice having the first transition metal in the core of the crystal lattice and the second transition metal atom on the periphery of the crystal lattice. wherein the precursor materials are selected from the group consisting of iron citrate, magnesium chloride, tetra-alkyl silicate, cobalt nitrate, and manganese nitrate, wherein the method is such that alkaline earth metal-silicates formed from the tetra-alkyl silicate and the magnesium chloride, are first introduced into an aqueous media in the form of a sol, the iron from iron citrate acts as a chelating agent in the core of the silica-alkaline earth metal sol, the second or more metal precursors, which are the cobalt nitrate and the manganese nitrate are added to organize them at the periphery of a formed framework crystal lattice, wherein a precipitating agent is added such that water is removed to produce a Fe-cored alkaline earth metal-silicate which has precursor material from the first transition material deposited around the framework producing a solid material containing single crystals inside the solid.

A third transition metal can be added to the step (b) dispersion and then an alkaline solution can be added to precipitate a silica/alkaline-earth metal/transition metals agglomerate that includes the first transition metal core bound to a silica-alkaline earth metal framework crystal lattice and the second and third transition metals on the periphery of the framework crystal lattice and precursor material. Prior to step (c), the precipitated material can be isolated and dried at a temperature of 100 C to 150 °C, preferably 130 °C. The dried material can be calcined at a temperature of 300 °C to 550 °C, preferably 450 °C. Step (b) precipitation can include adding an alkaline solution comprising ammonia, preferably 7 M ammonia to the solution.

In yet another aspect of the present invention, the use of the bulk-metal crystalline catalyst in a method of producing olefins from synthesis gas are described. A method can include contacting a reactant feed that includes hydrogen (H₂) and carbon monoxide (CO) with the catalyst(s) of the present invention, or made by the methods of the present invention, under conditions sufficient to produce an olefin. Conditions can include temperature (*e*.*g*.*,* 230 °C to 400 °C, preferably, 240 °C to 350 °C), weighted hourly space velocity (WHSV) *(e.g.,* 1000 h⁻¹ to 3000 h⁻¹, preferably 1500 h⁻¹ to 2000 h⁻¹), pressure (*e.g.,* 0.1 MPa to 1 MPa, preferably 0.5 MPa), or combinations thereof. A molar ratio of H₂ to CO can be 1:1 to 10:1, preferably 2:1. The olefin selectivity of the catalyst can be at least 15 mol.%, preferably 20 mol.%, the olefin conversion can be at least 30 mol.%, preferably at least 70 mol.%, more preferably at least 90 mol.%, and the methane selectivity can be less than 30 mol.%, or combinations thereof.

The following includes definitions of various terms and phrases used throughout this specification.

An alkyl group is linear or branched, substituted or substituted, saturated hydrocarbon. Non-limiting examples of alkyl group substituents include alkyl, halogen, hydroxyl, alkyloxy, haloalkyl, haloalkoxy, carboxylic acid, ester, amine, amide, nitrile, acyl, thiol and thioether.

The terms "about" or "approximately" are defined as being close to as understood by one of ordinary skill in the art. In one non-limiting embodiment, the terms are defined to be within 10%, preferably within 5%, more preferably within 1%, and most preferably within 0.5%.

The terms "wt.%", "vol.%", or "mol.%" refers to a weight percentage of a component, a volume percentage of a component, or molar percentage of a component, respectively, based on the total weight, the total volume of material, or total moles, that includes the component. In a non-limiting example, 10 grams of component in 100 grams of the material is 10 wt.% of component.

The term "bulk metal crystalline catalyst" as that term is used in the specification and/or claims, means that the catalyst includes one metal, and does not require a carrier or a support.

The term "substantially" and its variations are defined to include ranges within 10%, within 5%, within 1%, or within 0.5%.

The terms "inhibiting" or "reducing" or "preventing" or "avoiding" or any variation of these terms, when used in the claims and/or the specification includes any measurable decrease or complete inhibition to achieve a desired result.

The term "effective," as that term is used in the specification and/or claims, means adequate to accomplish a desired, expected, or intended result.

The use of the words "a" or "an" when used in conjunction with any of the terms "comprising," "including," "containing," or "having" in the claims, or the specification, may mean "one," but it is also consistent with the meaning of "one or more," "at least one," and "one or more than one."

The words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The catalysts of the present invention can "comprise," "consist essentially of," or "consist of' particular ingredients, components, compositions, *etc.* disclosed throughout the specification. With respect to the transitional phrase "consisting essentially of," in one non-limiting aspect, a basic and novel characteristic of the catalysts of the present invention are their abilities to catalyze production of olefins from synthesis gas.

The bulk-metal crystalline catalyst of the invention comprises a first transition metal core surrounded by a silica-alkaline earth metal framework crystal lattice and including at least two transition metal atoms bound to the periphery of the framework crystal lattice, wherein the first transition metal core comprises iron (Fe) and wherein the catalyst has a formula of (Ml)a(M2)b(Fe)c(Si)x(M3)yOz , where M1 and M2 are the transitions metals selected from the group consisting of cobalt, ruthenium, rhodium, manganese, and combinations thereof, M3 is an alkaline earth metal, and 0.01 ≤ a < 1, 0.07 ≤ b <1, 0.01 ≤ c < 1, 0.03 ≤ x < 1, 0.26

Preferably, the alkaline earth metal (M3 ) is selected from the group consisting of magnesium, calcium, strontium, barium or oxides and mixtures thereof.

More preferably, the alkaline earth metal is magnesium.

More preferably, the catalyst has a formula of (Mn)a(Co)b(Fe)c(Si)x(Mg)yOz where 0.01 ≤ a < 1, 0.07 < b <1, 0.01 ≤ c < 1, 0.03 ≤ x < 1, 0.26 < y < 1, and z is balances the valence of the catalyst. More preferably, the catalyst is absent a binder.

The method for preparing the bulk metal crystalline catalyst of the invention comprises the steps:
(a) obtaining a solution of a silicon precursor material, an alkaline earth metal precursor material and at least two transition metal precursor materials;
(b) precipitating a silica/alkaline-earth metal/transition metals agglomerate from the solution, the silica/ alkaline-earth metal/transition metals agglomerate comprising the first transition metal core bound to a silica-alkaline earth metal framework crystal lattice and the second transition metal on the periphery of the framework crystal lattice and precursor material; and
(c) contacting the precipitated material with an oxidizing agent to remove the precursor material and produce a bulk metal crystalline catalyst having a silica-alkaline earth metal crystal lattice having the first transition metal in the core of the crystal lattice and the second transition metal atom on the periphery of the crystal lattice,

wherein the precursor materials are selected from the group consisting of iron citrate, magnesium chloride, tetra-alkyl silicate, cobalt nitrate, and manganese nitrate,
wherein the method is such that alkaline earth metal-silicates formed from the tetra-alkyl silicate and the magnesium chloride, are first introduced into an aqueous media in the form of a sol,
the iron from iron citrate acts as a chelating agent in the core of the silica-alkaline earth metal sol, the second or more metal precursors, which are the cobalt nitrate and the manganese nitrate are added to organize them at the periphery of a formed framework crystal lattice,
wherein a precipitating agent is added such that water is removed to produce a Fe-cored alkaline earth metal-silicate which has precursor material from the first transition material deposited around the framework producing a solid material containing single crystals inside the solid.

Preferably, the method further comprises adding a third transition metal to the step (b) dispersion and then adding an alkaline solution to precipitate a silica/alkaline-earth metal/transition metals agglomerate comprising the first transition metal core bound to a silica-alkaline earth metal framework crystal lattice and the second and third transition metals on the periphery of the framework crystal lattice and precursor material.

More preferably, the method further comprises isolating and drying the precipitated material at a temperature of 100 C to 150 °C, preferably 130 °C prior to step (c).

More preferably, the method further comprises isolating and drying the crystalline material of step (c) 100 °C to 150 °C, preferably 130 °C.

More preferably, the method comprises calcining the dried material at 300 °C to 550 °C, preferably 450 °C.

More preferably, the method comprises adding an alkaline solution comprising ammonia, preferably 7 M ammonia to the solution, the oxidizing solution in step (c) hydrogen peroxide (H2O2), or both.

The use of the bulk-metal crystalline catalyst of the invention in a method of producing olefins from synthesis gas, comprises contacting a reactant feed comprising hydrogen (H2) and carbon monoxide (CO), under conditions sufficient to produce an olefin.

Preferably, the conditions comprise a temperature from 230 °C to 400 °C, preferably, 240 °C to 350 °C, a weighted hourly space velocity of 1000 h⁻¹ to 3000 h⁻¹, preferably 1200 h⁻¹ to 2000 h⁻¹ , a pressure of 0.1 to 1 MPa, preferably 0.5 MPa, or combinations thereof. More preferably, the molar ratio of H2 to CO is 1:1 to 10:1, preferably 2:1.

Other objects, features and advantages of the present invention will become apparent from the following figures, detailed description, and examples. It should be understood, however, that the figures, detailed description, and examples, while indicating specific embodiments of the invention, are given by way of illustration only and are not meant to be limiting.

### BRIEF DESCRIPTION OF THE DRAWINGS

Advantages of the present invention may become apparent to those skilled in the art with the benefit of the following detailed description and upon reference to the accompanying drawings.
**FIG. 1** depicts an image of a bimetallic bulk metal crystalline catalyst (CoFeSiMgO) of the present invention.
**FIG. 2** depicts an image of a trimetallic bulk metal crystalline catalyst (MnCoFeSiMgO) of the present invention.

While the invention is susceptible to various modifications and alternative forms, specific embodiments thereof are shown by way of example in the drawings. The drawings may not be to scale.

### DETAILED DESCRIPTION OF THE INVENTION

A discovery has been made that provides a solution to problems associated with catalysts used in the Fisher-Tropsch process to produce olefins from syngas. The discovery is premised on using a bulk bimetallic or multimetallic crystalline catalyst(s). The crystalline catalyst includes a first catalytic transition metal core surrounded by a silica-alkaline earth metal framework crystal lattice and including at least one additional catalytic transition metal bound to the periphery of the framework crystal lattice. Non-limiting examples of the transition metal are Fe, Co, Mn, Rh, Ru, and combinations thereof. The first transition metal is Fe and the transition metal(s) bound to the periphery of the framework are Co, Mn, Rh, Ru, or combinations thereof. Further, the catalytic activity and stability for the catalyst of the present invention is comparable or better as compared to the conventional catalysts for the Fischer-Tropsch process. Therefore, the catalyst of the present invention provides a technical solution to at least some of the problems associated with the currently available catalysts for the Fischer-Tropsch process mentioned above, such as low selectivity, low catalytic activity, and/or low stability.

These and other non-limiting aspects of the present invention are discussed in further detail in the following sections.

### A. Catalysts of the Present Invention

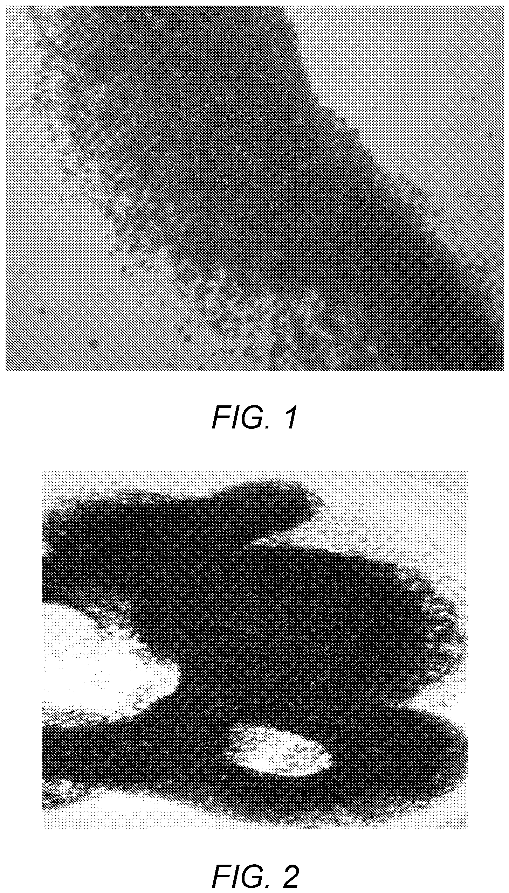

The catalyst of the present invention is a bulk metal crystalline catalyst. Said another way, the catalyst is not supported or does not include a binder. The catalyst can be a single crystal catalyst (See, for example, FIGS. 1 and 2 of the Examples). The catalyst can include a silica-alkaline earth metal crystal framework that includes at least two transition metals. Non-limiting examples of alkaline earth metals (Column 2 of the Periodic Table) include Mg, Ca, Sr, Ba and combinations thereof. Non-limiting examples of transition metals (Columns 5-12 of the Periodic Table include Mn, Fe, Co, Rh, Ru, and combinations thereof. The catalyst of the present invention can include up to 20 wt. % of the total amount of transition metal, from 0.001 wt.% to 20 wt. %, from 0.01 wt. % to 15 wt. %, or from 1 wt. % to 10 wt. % and all wt.% or at least, equal to, or between any two of 0.001 wt.%, 0.01 wt.%, 0.1 wt.%, 1 wt.%, 2 wt.%, 3 wt.%, 4 wt.%, 5 wt.%, 6 wt.%, 7 wt.%, 8 wt.%, 9 wt.%, 10 wt.%, 15 wt.%, and 20 wt.%, from 0.001 to 1 wt.% alkaline earth metal, with the balance being silicon and oxygen.

The catalyst has a formula of (M¹)*ₐ*(Fe)*_{c}*Si*ₓ*M³*_{y}*O*_{z}* where M¹ is a transition metal selected from the group consisting of cobalt, ruthenium, rhodium manganese, and combinations thereof M³ is an alkaline earth metal, and 0.01 ≤ *a* < 1 or at least, equal to, or between any two of 0.01, 0.05, 0.1, 0.2, 0.3, 0.4, 0.5, 0.6, 0.7, 0.8, 0.9 and 1; 0.01≤ *c* < 1, or at least, equal to, or between any two of 0.01, 0.05, 0.1, 0.2, 0.3, 0.4, 0.5, 0.6, 0.7, 0.8, 0.9 and 1; 0.03 ≤ *x* < 1, or at least, equal to, or between any two of 0.3, 0.4, 0.5, 0.6, 0.7, 0.8, 0.9 and 1; 0.26 ≤ *y* < 1, or at least, equal to, or between any two of 0.26, 0.3, 0.4, 0.5, 0.6, 0.7, 0.8, 0.9 and 1; and z is balances the valence of the catalyst. In some embodiments, z is 0.30 to 0.40, or 0.35 to 0.36, or 0.35 to 0.36.

In some instances, the catalyst can include at least two transitions metals and have a formula of (M¹)*ₐ*(M²)*_{b}*(Fe)*_{c}*Si*ₓ*M³*_{y}*O*_{z}* where M¹ and M² are the at least two transition metals selected from the group consisting of cobalt, ruthenium, rhodium manganese, and combinations thereof M³ is an alkaline earth metal, and 0.01 ≤ *a* < 1, or at least, equal to, or between any two of 0.01, 0.05, 0.1, 0.2, 0.3, 0.4, 0.5, 0.6, 0.7, 0.8, 0.9 and 1; 0.07 ≤ *b* <1, or at least, equal to, or between any two of 0.7, 0.8, 0.9 and 1; 0.01 ≤ *c* < 1, or at least, equal to, or between any two of 0.01, 0.05, 0.1, 0.2, 0.3, 0.4, 0.5, 0.6, 0.7, 0.8, 0.9 and 1; 0.03 ≤ *x* < 1, or at least, equal to, or between any two of 0.3, 0.4, 0.5, 0.6, 0.7, 0.8, 0.9 and 1; 0.26 < *y* ≤ 1, or at least, equal to, or between any two of 0.26, 0.3, 0.4, 0.5, 0.6, 0.7, 0.8, 0.9 and 1; and z is balances the valence of the catalyst. In some embodiments, z is 0.30 to 0.40, or 0.35 to 0.36.

In some embodiments, the catalyst is (consists of) a MnCoFeSiMgO bulk metal crystalline catalyst. Such a catalyst can have a formula of (Mn)*ₐ*(Co)_{b}FeSiₓ(Mg)*_{y}*O*_{z}* where 0.01 ≤ *a* < 1, or at least, equal to, or between any two of 0.01, 0.05, 0.1, 0.2, 0.3, 0.4, 0.5, 0.6, 0.7, 0.8, 0.9 and 1; 0.07 ≤ *b* <1, or at least, equal to, or between any two of 0.7, 0.8, 0.9 and 1; 0.01 ≤ *c* < 1, or at least, equal to, or between any two of 0.01, 0.05, 0.1, 0.2, 0.3, 0.4, 0.5, 0.6, 0.7, 0.8, 0.9 and 1; 0.03 ≤ *x* < 1, or at least, equal to, or between any two of 0.3, 0.4, 0.5, 0.6, 0.7, 0.8, 0.9 and 1; 0.26 < *y* ≤ 1, or at least, equal to, or between any two of 0.26, 0.3, 0.4, 0.5, 0.6, 0.7, 0.8, 0.9 and 1; and *z* is balances the valence of the catalyst. In some embodiments, z is 0.30 to 0.40, or 0.35 to 0.36.

In some embodiments, the active catalyst can include a molar ratio of cobalt to manganese in a range of 0.05 to 3 and all ranges and values there between including 0.05 to 0.10, 0.10 to 0.20, 0.20 to 0.40, 0.40 to 0.60, 0.60 to 0.80, 0.80 to 1.0, 1.0 to 1.2, 1.2 to 1.4, 1.4 to 1.6, 1.6 to 1.8, 1.8 to 2.0, 2.0 to 2.2, 2.2 to 2.4, 2.4 to 2.6, 2.6 to 2.8, and 2.8 to 3.0. A weight ratio of active metal (cobalt and manganese) to silica (SiO) may be in a range of 0.05 to 5 and all ranges and values there between including 0.05 to 0.10, 0.10 to 0.20, 0.20 to 0.40, 0.40 to 0.60, 0.60 to 0.80, 0.80 to 1.0, 1.0 to 1.2, 1.2 to 1.4, 1.4 to 1.6, 1.6 to 1.8, 1.8 to 2.0, 2.0 to 2.2, 2.2 to 2.4, 2.4 to 2.6, 2.6 to 2.8, 2.8 to 3.0, 3.0 to 3.2, 3.2 to 3.4, 3.4 to 3.6, 3.6 to 3.8, 3.8 to 4.0, 4.0 to 4.2, 4.2 to 4.4, 4.4 to 4.6, 4.6 to 4.8, and 4.8 to 5.0. Overall, the active catalyst may have a composition of 3 to 20 wt.% manganese, 0.05 to 8 wt.% cobalt, 40 to 80 wt.% silica, and 0.05 to 8 wt. % iron. Stability of the active catalyst can be quantified at a conversion rate of 30 to 90 over 100 hours under a temperature of 240 to 350 °C.

### B. Preparation of the Bulk Metal Crystalline Catalysts of the Present Invention

The bulk metal crystalline catalysts of the present invention are made according to claim 6 using methodology to produce singularly crystalline transition metal based alkaline earth-silicates. the method is such that alkaline earth metal-silicates formed from the tetra-alkyl silicate and the magnesium chloride, are first introduced into an aqueous media in the form of a sol, the iron from iron citrate acts as a chelating agent in the core of the silica-alkaline earth metal sol, the second or more metal precursors, which are the cobalt nitrate and the manganese nitrate are added to organize them at the periphery of a formed framework crystal lattice, wherein a precipitating agent is added such that water is removed to produce a Fe-cored alkaline earth metal-silicate which has precursor material from the first transition material deposited around the framework producing a solid material containing single crystals inside the solid. To release (rejuvenate) the crystals, the precursor material is removed *via* oxidation of the precursor (*e.g.,* washing with an oxidizing agent) to produce a distinct single crystal bulk metal catalyst. This methodology is in contrast to methods using hydrophilic silica as a binder and/or fumed silica as a support.

According to embodiments of the invention, a method may include providing an alkaline earth metal precursor solution. The alkaline earth metal precursor is magnesium chloride, and the solvent can be water. The alkaline earth metal salt solution may have a concentration in a range of 0.1 to 5 M and all ranges and values there between including 0.1 to 0.2 M, 0.2 to 0.4 M, 0.4 to 0.6 M, 0.6 to 0.8 M, 0.8 to 1.0 M, 1.0 to 1.2 M, 1.2 to 1.4 M, 1.4 to 1.6 M, 1.6 to 1.8 M, 1.8 to 2.0 M, 2.0 to 2.2 M, 2.2 to 2.4 M, 2.4 to 2.6 M, 2.6 to 2.8 M, 2.8 to 3.0 M, 3.0 to 3.2 M, 3.2 to 3.4 M, 3.4 to 3.6 M, 3.6 to 3.8 M, 3.8 to 4.0 M, 4.0 to 4.2 M, 4.2 to 4.4 M, 4.4 to 4.6 M, 4.6 to 4.8 M, and 4.8 to 5.0 M. In embodiments of the invention, the alkaline metal salt solution may be continuously stirred under a temperature in a range of 45 °C to 90 °C and all ranges and values there between. The duration for stirring may be in a range of 1 to 5 hours and all ranges and values there between.

A silica precursor material can be added to the alkaline earth metal (M³) solution. In some embodiments, the silica precursor material is added slowly (*e.g.,* dropwise over time). The silica precursor material is tetra-alkyl silicate, diethoxydimethylsilane (DEMS), tetramethyl orthosilicate (TMOS), tetraethyl orthosilicate (TEOS), and combinations thereof. In embodiments of the invention, the tetra-alkyl silicate can be TEOS. According to embodiments of the invention, the first mixture may have a alkaline earth metal to silicon weight (*e.g.,* M³ :Si) ratio of 0.3 to 2 and all ranges and values there between including 0.4, 0.5, 0.6, 0.7, 0.8, 0.9, 1.0, 1.1, 1.2, 1.3, 1.4, 1.5, 1.6, 1.7, 1.8, 1.9, and 2.0.

The first transition metal precursor solution can be added to the solution (first mixture) followed by the second transition metal precursor. By way of example, an iron precursor material can be added to the alkaline metal precursor/silica precursor solution, followed by a cobalt precursor material to form a second mixture. The iron precursor is ferric citrate. The cobalt precursor is cobalt nitrate. In some embodiments, the amount of iron precursor added to the second mixture can be 0.1 to 5 g and all ranges and values there between including 0.1 to 0.5 g, 0.5 to 1.0 g, 1.0 to 1.5 g, 1.5 to 2.0 g, 2.0 to 2.5 g, 2.5 to 3.0 g, 3.0 to 3.5 g, 3.5 to 4.0 g, 4.0 to 4.5 g, 4.5 to 5.0 g. In embodiments of the invention, amount of cobalt precursor material added to the second mixture can be 0.1 to 5 g and all ranges and values there between including 0.1 to 0.5 g, 0.5 to 1.0 g, 1.0 to 1.5 g, 1.5 to 2.0 g, 2.0 to 2.5 g, 2.5 to 3.0 g, 3.0 to 3.5 g, 3.5 to 4.0 g, 4.0 to 4.5 g, 4.5 to 5.0 g. In embodiments of the invention, the second mixture can be stirred for a duration of 0.3 to 5 hrs and all ranges and values there between including 0.3 to 0.5 hrs, 0.5 to 1.0 hrs, 1.0 to 1.5 hrs, 1.5 to 2.0 hrs, 2.0 to 2.5 hrs, 2.5 to 3.0 hrs, 3.0 to 3.5 hrs, 3.5 to 4.0 hrs, 4.0 to 4.5 hrs, 4.5 to 5.0 hrs at a temperature of 20 to 30 °C, or about 25 °C.

A precipitating agent can be added to the second mixture to form a third mixture. A non-limiting example of the precipitating agent may include ammonia (e.g., 1 to 8 M, or 1, 2, 3, 4, 5, 6, 7, 8 M). In embodiments of the invention, the amount of the precipitating agent added to the third mixture may be in a range of 45 to 100 mL, or and all ranges and values there between, including 45, 50, 55, 60, 65, 70, 75, 80, 85, 90, 95 and 100 mL. The third mixture may be continuously stirred for a duration of 0.5 to 5 hrs including 1, hrs, 2 hrs, 3 hrs, 4 hrs, and 5 hrs at a temperature of 20 to 30 °C, or about 25 °C until a gel is obtained. The composition of the third mixture may include 1, 2, 3, 4, 5, 6, 7, or 8 wt.% cobalt, 0.5, 1, 1.5 or 2 wt.% iron, 20, 21, 22, 23, 24, 25, 26, 27, 28, 29 or 30 wt.% magnesium, and 60 to 78.5 wt.% silica and all ranges and values there between.

In embodiments, where an additional transition metal *(e.g.,* a third, fourth, fifth, *etc.* transition metal) is desired, the additional transition metal precursor(s) can be added to the third mixture to form a fourth mixture. Non-limiting examples of a third transition metal precursor(s) include nitrates, acetates, and combinations thereof of the additional transition metal(s). In some embodiments, the additional transition metal precursor(s) are a magnesium precursor material, a rhodium precursor material, or a ruthenium precursor material. An amount of additional transition metal(s) in the fourth mixture can be 0.1 to 5 g and all ranges and values there between including 0.1 to 0.5 g, 0.5 to 1.0 g, 1.0 to 1.5 g, 1.5 to 2.0 g, 2.0 to 2.5 g, 2.5 to 3.0 g, 3.0 to 3.5 g, 3.5 to 4.0 g, 4.0 to 4.5 g, 4.5 to 5.0 g. The fourth mixture can be agitated at a temperature of 20 to 30 °C, or about 25 °C for 0.5 to 5 hrs including 0.5 to 1.0 hrs, 1.0 to 1.5 hrs, 1.5 to 2.0 hrs, 2.0 to 2.5 hrs, 2.5 to 3.0 hrs, 3.0 to 3.5 hrs, 3.5 to 4.0 hrs, 4.0 to 4.5 hrs, 4.5 to 5.0 hrs.

A second quantity of the precipitating agent can be added to the fourth mixture to form a fifth mixture and stirring the fifth mixture until a gel is obtained. According to embodiments of the invention, the second quantity may be 10 to 50 ml of the ammonia solution (1 to 10 M) in the fifth mixture. A fifth mixture can be stirred at a temperature of 20 to 30 °C, or about 25 °C for 0.5 to 5 hrs including 0.5 to 1.0 hrs, 1.0 to 1.5 hrs, 1.5 to 2.0 hrs, 2.0 to 2.5 hrs, 2.5 to 3.0 hrs, 3.0 to 3.5 hrs, 3.5 to 4.0 hrs, 4.0 to 4.5 hrs, 4.5 to 5.0 hrs. The composition of the fifth mixture may include 1, 2, 3, 4, 5, 6, 7, or 8 wt.% cobalt, 6, 6.5, 7, 7.5 or 8 % manganese, 0.5, 1, 1.5, or wt.% iron, 20, 21, 22, 23, 24, 25, 26, 27, 28, 29, or 30 wt.% magnesium, and 52 to 82.5 wt.% silica.

According to embodiments of the invention, the gel from the third mixture and/or second mixture can be isolated (*e.g.,* filtered or centrifuged), washed with hot water to remove the ammonia, and dried. In embodiments of the invention, the gel may be dried at a temperature of 100 to 150 °C and all values and ranges there between including 100 to 105 °C, 105 to 110 °C, 110 to 115 °C, 115 to 120 °C, 120 to 125 °C, 125 to 130 °C, 130 to 135 °C, 135 to 140 °C, 140 to 145 °C, and 145 to 150 °C. The drying process may be 5 to 12 hrs and all ranges and values there between including 6 hrs, 7 hrs, 8 hrs, 9 hrs, 10 hrs, 11 hrs. The dried gel can be contacted with an oxidizing solution *(e.g.,* 50 mL of 10% H₂O₂). In some embodiments, contacting includes immersing the dried gel in an oxidizing solution. Contacting the dried gel with the oxidizing solution removes the remaining precursor material(s) and provides single crystals of the catalytic material.

More preferably, the method comprises calcining the dried material at 300 °C to 550 °C, preferably 450 °C A heating rate for the calcination may be in a range of 1 to 5 °C/min and all ranges and values there between including 2 °C/min, 3 °C/min, and 4 °C/min. A calcination duration may be in a range of 2 to 12 hrs and all ranges and values there between including 3 hrs, 4 hrs, 5 hrs, 6 hrs, 7 hrs, 8 hrs, 9 hrs, 10 hrs, and 11 hrs.

### C. Method of Producing Olefins from a Reactant Feed that Includes H₂ and CO.

The active catalyst of the present invention can catalyze the conversion of a reactant feed that includes H₂ and CO (*e.g.,* synthesis gas) to produce olefins. Olefins can include olefins have 2, 3, 4, and 5 carbon atoms. For example, C2 to C4 olefins includes hydrocarbons that include 2, 3, 4 carbon atoms. Non-limiting examples of olefins include acetylene, propene, 1-butene, isobutylene, isoprene, and the like.

In embodiments of the invention, the synthesis gas can include 60 to 72 vol.% hydrogen and 28 to 40 vol.% carbon monoxide. In some embodiments, the molar ratio of H₂ to CO can be 1:1 to 10:1, or at least, equal to, or between any two of 1:1, 2:1, 3:1, 4:1, 5:1, 6:1, 7:1, 8:1, 9:1, and 10:1. The reaction conditions can include temperature, pressure and WHSV. The reaction temperature is in the range of 230 °C to 400 °C and all ranges and values there between including 240 to 250 °C, 250 to 260 °C, 260 to 270 °C, 270 to 280 °C, 280 to 290 °C, 290 to 300 °C, 300 to 310 °C, 310 to 320 °C, 320 to 330 °C, 330 to 340 °C, 340 to 350 °C, 350 to 360 °C, 360 to 370 °C, 370 to 380 °C, 380 to 390 °C, and 390 to 400 °C. The reaction conditions can include a reaction pressure in a range of 0.1 to 1.0 MPa and all ranges and values there between including 0.2, 0.3, 0.4, 0.5, 0.6, 0.7, 0.8 and 0.9 MPa. In embodiments of the invention, a weight hourly space velocity for the synthesis gas can a range of 1000 to 3000 hr⁻¹, and all ranges and values there between 1200 to 1300 hr⁻¹, 1300 to 1400 hr⁻¹, 1400 to 1500 hr⁻¹, 1500 to 1600 hr⁻¹, 1600 to 1700 hr⁻¹, 1700 to 1800 hr⁻¹, 1800 to 1900 hr⁻¹, 1900 to 2000 hr⁻¹, 2000 to 2100 hr⁻¹, 2100 to 2200 hr⁻¹, 2200 to 2300 hr⁻¹, 2300 to 2400 hr⁻¹, and 2400 to 2500 hr⁻¹. Contact of the reactant gas feed with the catalyst produces a product stream that includes olefins, C2+ paraffins, methane, and carbon dioxide (CO₂) can also be formed. The olefins can include C2 to C4 olefins. The product stream can be separated to produce a C2-C4 olefins stream and a by-product stream. The by-product stream can include paraffins, higher olefins (C5+ olefins), methane, and CO₂. Separation methods include distillation, membrane separations and the like, which are known in the art.

A conversion rate of the synthesis gas can be at least 30 to 100%, or at least, equal to, or between any two of 30%, 35%, 40%, 50%, 55%, 60%, 65%, 70%, 75%, 80%, 85%, 90%, 95% and 100%. Olefins selectivity can range from 10 to 100, or at least, equal to, or between any two of 10, 15, 20, 25, 30, 35, 40, 45, 50, 55, 60, 65, 70, 75, 80, 85, 90, and 100. C2 to C4 olefins selectivity can range from 10 to 100, or at least, equal to, or between any two of 10, 15, 20, 25, 30, 35, 40, 45, 50, 55, 60, 65, 70, 75, 80, 85, 90, and 100. The methane selectivity can be less than, equal to, or between any two of 30%, 25%, 20%, 15%, 10%, 5%, 1%, and 0%. In some instances, the total olefins selectivity at 240 °C is at least 30%, with the selectivity to C2 to C4 olefins being 20% and the methane selectivity being less than 15% after 413 hours on stream.

The method can also include activating the catalyst prior to contact with the reactant feed. To activate the catalyst a gas stream including a reducing agent (*e.g.,* hydrogen) and a chemically inert gas *(e.g.,* nitrogen) can be contacted with the catalyst (*e.g.,* flow through the catalyst bed) at a temperature of 300 to 350 °C and all ranges and values there between. A molar ratio for reducing gas to inert gas in the gas stream can be about 1:1. A heating rate for the activation may be in a range of 2 to 5 °C/min and all ranges and values there between including 3 °C /min and 4 °C /min. A weight hourly space velocity for the gas stream containing the reducing gas may be in a range of 3200 to 4000 hr⁻¹ and all ranges and values there between including 3200 to 3250 hr⁻¹, 3250 to 3300 hr⁻¹, 3300 to 3350 hr⁻¹, 3350 to 3400 hr⁻¹, 3400 to 3450 hr⁻¹, 3450 to 3500 hr⁻¹, 3500 to 3550 hr⁻¹, 3550 to 3600 hr⁻¹, 3600 to 3650 hr⁻¹, 3650 to 3700 hr⁻¹, 3700 to 3750 hr⁻¹, 3750 to 3800 hr⁻¹, 3800 to 3850 hr⁻¹, 3850 to 3900 hr⁻¹, 3900 to 3950 hr⁻¹, and 3950 to 4000 hr⁻¹.

An apparatus can be adapted for conversion of synthesis gas to C2 to C4 olefins using the active catalyst of the invention. The apparatus can include a fixed-bed flow reactor. The apparatus can include a catalyst bed in a fixed-bed flow reactor. The apparatus can also include a housing for containing the catalyst bed. In some embodiments the apparatus can include inlet means for introducing synthesis gas to the catalyst bed. The inlet means can an entrance adapted to receive synthesis gas. Further the apparatus can include an outlet means for removing the product stream comprising C2 to C4 olefins from the apparatus. The outlet means can include an exit adapted to flow the product stream from the housing. The apparatus can include the catalyst according to embodiments of the invention disposed in the catalyst bed. The apparatus may be a fluidized bed reactor, and/or a slurry reactor.

### EXAMPLES

The present invention will be described in greater detail by way of specific examples. The following examples are offered for illustrative purposes only, and are not intended to limit the invention in any manner.

### Materials Used

The following lab grade chemicals were used without further purification: manganese (II) nitrate tetrahydrate (>97% purity, SigmaMillipore), cobalt (II) nitrate hexahydrate (>98% purity, SigmaMillipore), magnesium chloride (1 M soln.), tetraethyl orthosilicate (TEOS), ferric citrate, ammonium hydroxide soln., and H₂O₂ soln.

### Example 1

### (Synthesis of Bulk Metal Crystalline CoFeSiMgO Catalysts)

Magnesium chloride (20 ml of 1 M) was diluted with distilled H₂O to 100 mL and stirred vigorously at 50 °C. Afterwards, TEOS (10.4 g) was added to the solution dropwise. Then, ferric citrate crystals (0.25 g) followed by cobalt nitrate (0.25 g) were added, and the mixture was stirred for 2 h. Ammonia (1 M, 100 mL solution) was added slowly to the mixture. The resultant solution was stirred for 2 h. The stirred solution was filtered and washed with hot water two times, and the resulting solid cake was placed in oven to dry overnight at 130 °C. After drying, the material was immersed in H₂O₂ (15 %, 50 mL) for 1 h, followed by filtration and drying for 4 h at 130 °C. The material was then calcined at 450 °C/3 °C min⁻¹ for 5 h in air until ready to be tested. Further samples were prepared by using 5 and 7 M ammonia solution. From hereafter, the samples are denoted by symbols "A, B C" for 1, 5 and 7 M ammonia solutions respectively. FIG. 1 depicts an image of the A (CoFeSiMgO) catalyst. The platelet type crystalline morphology is evident in FIG. 1.

### Example 2

### (Synthesis of Bulk Metal Crystalline CoMnFeSiMgO Catalysts)

Magnesium chloride (20 ml of 1 M) was diluted with distilled H₂O to 100 mL and stirred vigorously at 50 °C. Afterwards, TEOS (10.4 g) was added to the solution dropwise. Then, ferric citrate crystals (0.25 g) followed by cobalt nitrate (0.25 g) were added, and the mixture was stirred for 2 h. Ammonia (1 M, 50 mL solution) was added slowly to the mixture. The resultant solution was stirred for 1 h. At this point, Mn salt (1.5 g) was added to the mixture and the solution is stirred for another 1 h followed by addition of ammonia (1 M, 50 mL solution), stirred for 1 h, before filtration and washing with hot water two times. The stirred solution was filtered and washed with hot water two times, and the resulting solid cake was placed in oven to dry overnight at 130 °C. After drying, the material was immersed in H₂O₂ (15 %, 50 mL) for 1 h, followed by filtration and drying for 4 h at 130 °C. The material was then calcined at 450 °C/3 °C min⁻¹ for 5 h in air until ready to be tested. Further samples were prepared by using 5 and 7 M ammonia solution. From hereafter, the samples are denoted by symbols "D, E, F" for 1, 5 and 7 M ammonia solutions respectively. FIG. 2 depicts an image of the D (MnCoFeSiMgO) catalyst. The platelet type crystalline morphology is evident in FIG. 2.

### Example 3

### (Synthesis of Bulk Metal Crystalline CoMnFeSiMgO Catalysts)

Magnesium chloride (20 ml of 1 M) was diluted with distilled H₂O to 100 mL and stirred vigorously at 50 °C. Afterwards, TEOS (10.4 g) was added to the solution dropwise. Then, ferric citrate crystals (0.25 g) followed by cobalt nitrate (0.25 g) were added, and the mixture was stirred for 2 h. Ammonia (1 M, 50 mL solution) was added slowly to the mixture. The resultant solution was stirred for 1 h. At this point, Mn salt (1.5 g) was added to the mixture and the solution is stirred for another 1 h followed by addition of ammonia (1 M, 50 mL solution), stirred for 1 h, before filtration and washing with hot water two times. The stirred solution was filtered and washed with hot water two times, and the solid cake was placed in oven to dry overnight at 130 °C. After drying, the material was immersed in H₂O₂ (10 % 50 ml) for 1 h followed by filtration and drying for 4 h at 130 °C. The dried material was then calcined at 450 °C/3 °C min⁻¹ for 5 h in air until ready to be tested. Further samples are prepared by using 5 and 7 M ammonia solution. From hereafter, the samples are denoted by symbols "G, H, I" for 1, 5 and 7 M ammonia solutions respectively.

### Example 4

### (Synthesis of Bulk Metal Crystalline CoFeSiMgO Catalysts)

Magnesium chloride (20 ml of 1 M) was diluted with distilled H₂O to 100 mL and stirred vigorously at 50 °C. Afterwards, TEOS (10.4 g) was added to the solution dropwise. Then, ferric citrate crystals (0.25 g) followed by cobalt nitrate (1.5 g) were added, and the mixture was stirred for 2 h. Ammonia (1 M, 100 mL solution) was added slowly to the mixture. The resultant solution was stirred for 2 h. The stirred solution was filtered and washed with hot water two times, and the solid cake was placed in oven to dry overnight at 130 °C. After drying, the material was immersed in H₂O₂ (15% 50 ml) for 1 h followed by filtration and drying for 4 h at 130 °C. The dried material was then calcined at 450 °C/3 °C min⁻¹ for 5 h in air until ready to be tested. Further samples are prepared by using 5 and 7 M ammonia solution. From hereafter, the samples are denoted by symbols "J, K, L" for 1, 5 and 7 M ammonia solutions respectively.

### Example 5

### (Synthesis of Bulk Metal Crystalline CoMnFeSiMgO Catalysts)

Magnesium chloride (20 ml of 1 M) was diluted with distilled H₂O to 100 mL and stirred vigorously at 50 °C. Afterwards, TEOS (10.4 g) was added to the solution dropwise. Then, ferric citrate crystals (0.25 g) followed by cobalt nitrate (1.5 g) were added, and the mixture was stirred for 2 h. Ammonia (1 M, 50 mL solution) was added slowly to the mixture. The resultant solution was stirred for 1 h. At this point, Mn salt (1.5 g) was added to the mixture and the solution is stirred for another 1 h followed by addition of ammonia (1 M, 50 mL solution), stirred for 1 h, before filtration and washing with hot water two times. The stirred solution was filtered and washed with hot water two times, and the solid cake was placed in oven to dry overnight at 130 °C. After drying, the material was immersed in H₂O₂ (10 % 50 ml) for 1 h followed by filtration and drying for 4 h at 130 °C. The dried material was then calcined at 450 °C/3 °C min⁻¹ for 5 h in air until ready to be tested. Further samples are prepared by using 5 and 7 M ammonia solution. From hereafter, the samples are denoted by symbols "M, N, O" for 1, 5 and 7 M ammonia solutions respectively.

### Example 6

### (Catalyst Activity/Selectivity Evaluation)

The catalysts from Example 1-5 were evaluated for the activity and selectivity calculations along with short term as well as long studies of the catalyst stabilities. Prior to activity measurement, all of the catalysts were subjected to activation procedure which was performed at 350 °C with the ramp rate of 3 °C min⁻¹ for 16 h in 50:50 H₂/N₂ flow (WHSV: 3600 h⁻¹). Catalytic evaluation was carried out in high throughput fixed bed flow reactor setup housed in temperature controlled system fitted with regulators to maintain pressure during the reaction. The products of the reactions were analyzed through online GC analysis using an Agilent GC (Agilent Scientific Instruments, U.S.A.) with a capillary column equipped with TCD and FID detectors. The evaluation was carried out under the following conditions unless otherwise mentioned elsewhere; 5 bar, WHSV: 1875 h-1, H2/CO ratio of 2. The mass balance of the reactions is calculated to be 95 ± 5%.

The catalysts all performed the same and representative results for catalyst E (CoMnFeSiMgO) are presented in Table 1. Final catalytic materials were crystalline in nature as shown in FIGS. 1 and 2, the crystal including a silica-magnesia support framework with iron at the core and cobalt and manganese on the periphery. The catalytic materials contain the properties of both cobalt and iron, as catalysts were stable for very long time durations like cobalt and have high WGS activity like iron based catalysts. This was considered to be single crystal based catalysis as the materials have crystalline structures. While representative images of two catalysts are depicted, all the catalysts were crystalline in nature. The catalysts were tested for more than 400 h with no signs of deactivation. A maximum of 90 % conversion of syngas was achieved with 20 % olefins selectivity with only 5 % C5+ in it. The same trend is seen in paraffin selectivity. The testing was done in a range of 240-350 °C temperature. The WGS activity was found to be high at high temperatures with high levels of CO₂ and CH₄. As the temperature lowered, CO₂ and CH₄ was decreased due to low WGS activity.

**Table 1**

| | | | | | | |
|---|---|---|---|---|---|---|
| **Temperature [°C]** | **350** | **325** | **300** | **280** | **260** | **240** |
| **Hours** | **193** | **242** | **266** | **338** | **367** | **417** |
| **Conversion (mol. %)** | **84** | **86** | **76** | **63** | **41** | **18** |

| | Selectivities (mol. %) | | | | | |
|---|---|---|---|---|---|---|
| **Olefins** | 27 | 27 | 28 | 29 | 34 | 33 |
| **Paraffins** | 14 | 17 | 19 | 22 | 24 | 28 |
| **Methane** | 23 | 18 | 15 | 14 | 14 | 20 |
| **C₂-C₄** | 12 | 13 | 13 | 14 | 13 | 16 |
| **C₂=C₄** | 23 | 21 | 20 | 20 | 22 | 23 |
| **C₅₊-** | 3 | 5 | 7 | 10 | 15 | 12 |
| **C₅₊=** | 4 | 6 | 8 | 9 | 12 | 10 |
| **CO₂** | 36 | 37 | 36 | 32 | 23 | 17 |
| **Olefins (% yield)** | 22 | 22.3 | 20.9 | 18 | 13.9 | 5.8 |
| ^{∗}Catalysts were tested after activation under the following conditions: WHSV: 1875 h⁻¹, H₂/CO: 2. | | | | | | |

## Claims

1. A bulk-metal crystalline catalyst comprising a first transition metal core surrounded by a silica-alkaline earth metal framework crystal lattice and including at least two transition metal atoms bound to the periphery of the framework crystal lattice,
wherein the first transition metal core comprises iron (Fe) and wherein the catalyst has a formula of (M¹)*ₐ*(M²)*_{b}*(Fe)*_{c}*Si*ₓ*M³*_{y}*O*_{z}* where M¹ and M² are the transitions metals selected from the group consisting of cobalt, ruthenium, rhodium, manganese, and combinations thereof M³ is an alkaline earth metal, and 0.01 ≤ *a <* 1, 0.07 ≤ *b* <1, 0.01 ≤ *c* < 1, 0.03 ≤ *x* < 1, 0.26 < *y* ≤ 1, and z balances the valence of the catalyst,
or
wherein first transition metal core is Fe metal surrounded by a silica-magnesia framework crystal lattice and includes cobalt (Co) and manganese (Mn) atoms bound to periphery of the framework crystal lattice.

2. The bulk-metal crystalline catalyst of claim 1, wherein the alkaline earth metal (M³) is selected from the group consisting of magnesium, calcium, strontium, barium or oxides and mixtures thereof.

3. The bulk-metal crystalline catalyst of claim 2, wherein the alkaline earth metal is magnesium.

4. The bulk-metal crystalline catalyst of claim 1, wherein the catalyst has a formula of (Mn)*ₐ*(Co)*_{b}*Fe*_{c}*Siₓ(Mg)*_{y}*O*_{z}* where 0.01 ≤ *a <* 1, 0.07 ≤ *b <1,* 0.01 ≤ *c* < 1, 0.03 ≤ *x* < 1, 0.26 < *y* ≤ 1, and z is balances the valence of the catalyst.

5. The bulk-metal crystalline catalyst of any one of claims 1 to 4, wherein the catalyst is absent a binder.

6. A method for preparing the bulk metal crystalline catalyst of any one of claims 1 to 5, comprising the steps:
(a) obtaining a solution of a silicon precursor material, an alkaline earth metal precursor material and at least two transition metal precursor materials;
(b) precipitating a silica/alkaline-earth metal/transition metals agglomerate from the solution, the silica/alkaline-earth metal/transition metals agglomerate comprising the first transition metal core bound to a silica-alkaline earth metal framework crystal lattice and the second transition metal on the periphery of the framework crystal lattice and precursor material; and
(c) contacting the precipitated material with an oxidizing agent to remove the precursor material and produce a bulk metal crystalline catalyst having a silica-alkaline earth metal crystal lattice having the first transition metal in the core of the crystal lattice and the second transition metal atom on the periphery of the crystal lattice,
wherein the precursor materials are selected from the group consisting of iron citrate, magnesium chloride, tetra-alkyl silicate, cobalt nitrate, and manganese nitrate,
wherein the method is such that
alkaline earth metal-silicates formed from the tetra-alkyl silicate and the magnesium chloride, are first introduced into an aqueous media in the form of a sol,
the iron from iron citrate acts as a chelating agent in the core of the silica-alkaline earth metal sol,
the second or more metal precursors, which are the cobalt nitrate and the manganese nitrate are added to organize them at the periphery of a formed framework crystal lattice, wherein a precipitating agent is added such that water is removed to produce a Fe-cored alkaline earth metal-silicate which has precursor material from the first transition material deposited around the framework producing a solid material containing single crystals inside the solid.

7. The method of claim 6, further comprising adding a third transition metal to the step (b) dispersion and then adding an alkaline solution to precipitate a silica/alkaline-earth metal/transition metals agglomerate comprising the first transition metal core bound to a silica-alkaline earth metal framework crystal lattice and the second and third transition metals on the periphery of the framework crystal lattice and precursor material.

8. The method of any one of claims 6 and 7, further comprising isolating and drying the precipitated material at a temperature of 100 C to 150 °C, preferably 130 °C prior to step (c).

9. The method of any one of claims 6 to 8, further comprising isolating and drying the crystalline material of step (c) 100 C to 150 °C, preferably 130 °C.

10. The method of claim 9, further comprising calcining the dried material at 300 °C to 550 °C, preferably 450 °C.

11. The method of any one of claims 6 to 10, wherein step (b) comprising adding an alkaline solution comprising ammonia, preferably 7 M ammonia to the solution, the oxidizing solution in step (c) hydrogen peroxide (H₂O₂), or both.

12. Use of a bulk-metal crystalline catalyst of any one of claims 1 to 5 or a bulk-metal crystalline catalyst made by the method of any one of claims 6 to 11 in a method of producing olefins from synthesis gas, the method comprising contacting a reactant feed comprising hydrogen (H₂) and carbon monoxide (CO), under conditions sufficient to produce an olefin.

13. The use of claim 12, wherein the conditions comprise a temperature from 230 °C to 400 °C, preferably, 240 °C to 350 °C, a weighted hourly space velocity of 1000 h⁻¹ to 3000 h⁻¹, preferably 1200 h⁻¹ to 2000 h⁻¹, a pressure of 0.1 to 1 MPa, preferably 0.5 MPa, or combinations thereof.

14. The use of any one of claims 12 and 13, wherein a molar ratio of H₂ to CO is 1:1 to 10:1, preferably 2:1.

## Patentansprüche

1. Kristalliner Massenmetallkatalysator, umfassend einen ersten Übergangsmetallkern, der von einem Siliciumdioxid-Erdalkalimetall-Gerüstkristallgitter umgeben ist und mindestens zwei Übergangsmetallatome enthält, die an die Peripherie des Gerüstkristallgitters gebunden sind,
wobei der erste Übergangsmetallkern Eisen (Fe) umfasst und wobei der Katalysator die Formel (M¹)*ₐ*(M²)*_{b}*(Fe)*_{c}*Si*ₓ*M³*_{y}*O*_{z}* hat, wobei M¹ und M² die Übergangsmetalle sind, ausgewählt aus der Gruppe bestehend aus Kobalt, Ruthenium, Rhodium, Mangan und Kombinationen davon, M₃ ein Erdalkalimetall ist und 0,01 ≤ a < 1, 0,07 ≤ b <1, 0,01 ≤ c < 1, 0,03 ≤ x < 1, 0,26 < y ≤ 1, und z die Valenz des Katalysators ausgleicht,
oder
wobei der erste Übergangsmetallkern Fe-Metall ist, das von einem Siliciumdioxid-Magnesiumoxid-Gerüstkristallgitter umgeben ist und Kobalt- (Co) und Mangan- (Mn) Atome enthält, die an die Peripherie des Gerüstkristallgitters gebunden sind.

2. Kristalliner Massenmetallkatalysator nach Anspruch 1, wobei das Erdalkalimetall (M³) aus der Gruppe ausgewählt ist, die aus Magnesium, Calcium, Strontium, Barium oder Oxiden und Mischungen davon besteht.

3. Kristalliner Massenmetallkatalysator nach Anspruch 2, wobei das Erdalkalimetall Magnesium ist.

4. Kristalliner Massenmetall-Katalysator nach Anspruch 1, wobei der Katalysator die Formel (Mn)*ₐ*(Co)*_{b}*Fe*_{c}*Si*ₓ*(Mg)*_{y}*O*_{z}* hat, wobei 0,01 ≤ a < 1, 0,07 ≤ b <1, 0,01 ≤ c < 1, 0,03 ≤ x < 1, 0,26 < y ≤ 1 und z die Valenz des Katalysators ausgleicht.

5. Kristalliner Bulk-Metall-Katalysator nach einem der Ansprüche 1 bis 4, wobei der Katalysator kein Bindemittel enthält.

6. Verfahren zur Herstellung des kristallinen Massenmetall-Katalysators nach einem der Ansprüche 1 bis 5, umfassend die Schritte:
(a) Erhalten einer Lösung eines Siliziumvorläufermaterials, eines Erdalkalimetallvorläufermaterials und mindestens zweier Übergangsmetallvorläufermaterialien;
(b) Ausfällen eines Agglomerats aus Siliciumdioxid/Erdalkalimetall/Übergangsmetallen aus der Lösung, wobei das Agglomerat aus Siliciumdioxid/Erdalkalimetall/Übergangsmetallen den ersten Übergangsmetallkern, der an ein Siliciumdioxid-Erdalkalimetall-Gerüstkristallgitter gebunden ist, und das zweite Übergangsmetall an der Peripherie des Gerüstkristallgitters und Vorläufermaterial umfasst; und
(c) Inkontaktbringen des ausgefällten Materials mit einem Oxidationsmittel, um das Vorläufermaterial zu entfernen und einen massiven kristallinen Metallkatalysator mit einem Siliciumdioxid-Erdalkalimetall-Kristallgitter herzustellen, das das erste Übergangsmetall im Kern des Kristallgitters und das zweite Übergangsmetallatom an der Peripherie des Kristallgitters aufweist,
wobei die Vorläufermaterialien ausgewählt sind aus der Gruppe bestehend aus Eisencitrat, Magnesiumchlorid, Tetraalkylsilikat, Kobaltnitrat und Mangannitrat,
wobei das Verfahren derart ist, dass
Erdalkalimetallsilikate, die aus dem Tetraalkylsilikat und dem Magnesiumchlorid gebildet werden, zunächst in Form eines Sols in ein wässriges Medium eingebracht werden,
das Eisen aus dem Eisencitrat als Chelatbildner im Kern des Kieselsäure-Erdalkalimetall-Sols wirkt,
die zweiten oder mehreren Metallvorläufer, bei denen es sich um das Kobaltnitrat und das Mangannitrat handelt, zugegeben werden, um sie an der Peripherie eines gebildeten Gerüstkristallgitters anzuordnen, wobei ein Fällungsmittel zugegeben wird, so dass Wasser entfernt wird, um ein Fe-verkerntes Erdalkalimetallsilikat zu erzeugen, bei dem sich Vorläufermaterial aus dem ersten Übergangsmaterial um das Gerüst herum ablagert und ein festes Material erzeugt wird, das Einkristalle im Inneren des Feststoffs enthält.

7. Verfahren nach Anspruch 6, das ferner die Zugabe eines dritten Übergangsmetalls zu der Dispersion aus Schritt (b) und die anschließende Zugabe einer alkalischen Lösung umfasst, um ein Agglomerat aus Siliciumdioxid/Erdalkalimetall/Übergangsmetallen auszufällen, das den ersten Übergangsmetallkern, der an ein Siliciumdioxid-Erdalkalimetall-Gerüstkristallgitter gebunden ist, und das zweite und dritte Übergangsmetall an der Peripherie des Gerüstkristallgitters und das Vorläufermaterial umfasst.

8. Verfahren nach einem der Ansprüche 6 und 7, ferner umfassend das Isolieren und Trocknen des ausgefällten Materials bei einer Temperatur von 100 C bis 150 °C, vorzugsweise 130 °C, vor Schritt (c).

9. Verfahren nach einem der Ansprüche 6 bis 8, umfassend ferner das Isolieren und Trocknen des kristallinen Materials aus Schritt (c) bei 100 C bis 150 °C, vorzugsweise 130 °C.

10. Verfahren nach Anspruch 9, umfassend ferner das Kalzinieren des getrockneten Materials bei 300 °C bis 550 °C, vorzugsweise 450 °C.

11. Verfahren nach einem der Ansprüche 6 bis 10, wobei Schritt (b) die Zugabe einer alkalischen Lösung, die Ammoniak, vorzugsweise 7 M Ammoniak, enthält, zu der Lösung, der oxidierenden Lösung in Schritt (c), Wasserstoffperoxid (H₂O₂) oder beides umfasst.

12. Verwendung eines kristallinen Massenmetallkatalysators nach einem der Ansprüche 1 bis 5 oder eines kristallinen Massenmetallkatalysators, der nach dem Verfahren nach einem der Ansprüche 6 bis 11 hergestellt wurde, in einem Verfahren zur Herstellung von Olefinen aus Synthesegas, wobei das Verfahren das In-Kontakt-Bringen eines Reaktanten-Feeds, der Wasserstoff (H₂) und Kohlenmonoxid (CO) umfasst, unter Bedingungen umfasst, die geeignet sind, ein Olefin herzustellen.

13. Verwendung nach Anspruch 12, wobei die Bedingungen eine Temperatur von 230 °C bis 400 °C, vorzugsweise 240 °C bis 350 °C, eine gewichtete stündliche Raumgeschwindigkeit von 1000 h⁻¹ bis 3000 h⁻¹, vorzugsweise 1200 h⁻¹ bis 2000 h⁻¹, einen Druck von 0,1 bis 1 MPa, vorzugsweise 0,5 MPa, oder Kombinationen davon umfassen.

14. Verwendung nach einem der Ansprüche 12 und 13, wobei das molare Verhältnis von H₂ zu CO 1:1 bis 10:1, vorzugsweise 2:1, beträgt.

## Revendications

1. Catalyseur cristallin à base de métal en vrac comprenant un premier noyau de métal de transition entouré d'un réseau cristallin d'ossature de silice-métal alcalino-terreux et comprenant au moins deux atomes de métal de transition liés à la périphérie du réseau cristallin d'ossature,
dans lequel le premier noyau de métal de transition comprend du fer (Fe) et dans lequel le catalyseur a une formule de
(M¹) *ₐ* (M²) *_{b}* (Fe) *_{c}*Si*ₓ*M³*_{y}*O*_{z}* où M¹ et M² sont les métaux de transition choisis dans le groupe constitué par le cobalt, le ruthénium, le rhodium, le manganèse et leurs combinaisons M³ est un métal alcalino-terreux, et 0,01 ≤ a < 1,
0,07 ≤ b < 1, 0,01 ≤ c < 1, 0,03 ≤ x < 1, 0,26 < y ≤ 1, et z équilibre la valence du catalyseur,
ou
dans lequel le premier noyau de métal de transition est un métal Fe entouré d'un réseau cristallin d'ossature de silice-magnésie et comprend des atomes de cobalt (Co) et de manganèse (Mn) liés à la périphérie du réseau cristallin d'ossature.

2. Catalyseur cristallin à métal en vrac de la revendication 1, dans lequel le métal alcalino-terreux (M³) est choisi dans le groupe constitué par le magnésium, le calcium, le strontium, le baryum ou les oxydes et leurs mélanges.

3. Catalyseur cristallin à base de métal en vrac selon la revendication 2, dans lequel le métal alcalino-terreux est le magnésium.

4. Le catalyseur cristallin métallique en vrac de la revendication 1, dans lequel le catalyseur a une formule de (Mn) *ₐ* (Co) *_{b}*Fe*_{c}*Si*ₓ* (Mg) *_{y}*O*_{z}* où 0,01 ≤ a < 1, 0,07 ≤ b < 1, 0,01 ≤ c < 1, 0,03 ≤ x < 1, 0,26 < *y* ≤ 1, et z est le solde de la valence du catalyseur.

5. Le catalyseur cristallin en vrac métallique de l'une quelconque des revendications 1 à 4, dans lequel le catalyseur est absent d'un liant.

6. Procédé de préparation du catalyseur cristallin métallique en vrac de l'une quelconque des revendications 1 à 5, comprenant les étapes consistant à :
(a) obtenir une solution d'un matériau précurseur de silicium, d'un matériau précurseur de métal alcalino-terreux et d'au moins deux matériaux précurseurs de métal de transition ;
(b) précipiter un agglomérat de silice/métal alcalino-terreux/métaux de transition à partir de la solution, l'agglomérat de silice/métal alcalino-terreux/métaux de transition comprenant le premier noyau de métal de transition lié à un réseau cristallin d'ossature de silice-métal alcalino-terreux et le second métal de transition à la périphérie du réseau cristallin d'ossature et du matériau précurseur ; et
(c) la mise en contact du matériau précipité avec un agent oxydant pour éliminer le matériau précurseur et produire un catalyseur cristallin métallique en vrac ayant un réseau cristallin de silice-métal alcalino-terreux ayant le premier métal de transition dans le noyau du réseau cristallin et le second atome de métal de transition sur la périphérie du réseau cristallin,
dans lequel les matériaux précurseurs sont choisis dans le groupe constitué par le citrate de fer, le chlorure de magnésium, le silicate de tétra-alkyle, le nitrate de cobalt et le nitrate de manganèse,
dans lequel le procédé est tel que
les silicates de métaux alcalino-terreux formés à partir du silicate de tétra-alkyle et du chlorure de magnésium, sont d'abord introduits dans un milieu aqueux sous la forme d'un sol,
le fer provenant du citrate de fer agit comme un agent chélateur dans le noyau du sol de silice-métal alcalino-terreux,
le second ou plusieurs précurseurs métalliques, qui sont le nitrate de cobalt et le nitrate de manganèse, sont ajoutés pour les organiser à la périphérie d'un réseau cristallin d'ossature formé, dans lequel un agent de précipitation est ajouté de telle sorte que l'eau est éliminée pour produire un silicate de métal alcalino-terreux à noyau de Fe qui a un matériau précurseur du premier matériau de transition déposé autour de la charpente produisant un matériau solide contenant des monocristaux à l'intérieur du solide.

7. Procédé de la revendication 6, comprenant en outre l'addition d'un troisième métal de transition à la dispersion de l'étape (b), puis l'addition d'une solution alcaline pour précipiter un agglomérat de silice/métal alcalino-terreux/métaux de transition comprenant le premier noyau de métal de transition lié à un réseau cristallin d'ossature de silice-métal alcalino-terreux et les deuxième et troisième métaux de transition à la périphérie du réseau cristallin d'ossature et du matériau précurseur.

8. Procédé de l'une quelconque des revendications 6 et 7, comprenant en outre l'isolement et le séchage du matériau précipité à une température de 100 C à 150 °C, de préférence 130 °C avant l'étape (c).

9. Le procédé de l'une quelconque des revendications 6 à 8, comprenant en outre l'isolement et le séchage du matériau cristallin de l'étape (c) 100 C à 150 °C, de préférence 130 °C.

10. Procédé de la revendication 9, comprenant en outre la calcination du matériau séché à 300 °C à 550 °C, de préférence 450 °C.

11. Procédé de l'une quelconque des revendications 6 à 10, dans lequel l'étape (b) comprend l'ajout d'une solution alcaline comprenant de l'ammoniac, de préférence de l'ammoniac 7 M à la solution, la solution oxydante de l'étape (c) du peroxyde d'hydrogène (H₂O₂), ou les deux.

12. Utilisation d'un catalyseur cristallin métallique en vrac de l'une quelconque des revendications 1 à 5 ou d'un catalyseur cristallin métallique en vrac fabriqué par le procédé de l'une quelconque des revendications 6 à 11 dans un procédé de production d'oléfines à partir de gaz de synthèse, le procédé comprenant la mise en contact d'une charge de réactifs comprenant de l'hydrogène (H₂) et du monoxyde de carbone (CO), dans des conditions suffisantes pour produire une oléfine.

13. Utilisation de la revendication 12, dans laquelle les conditions comprennent une température de 230 °C à 400 °C, de préférence de 240 °C à 350 °C, une vitesse spatiale horaire pondérée de 1000 h⁻¹ à 3000 h⁻¹, de préférence de 1200 h⁻¹ à 2000 h⁻¹, une pression de 0,1 à 1 MPa, de préférence de 0,5 MPa, ou des combinaisons de celles-ci.

14. Utilisation de l'une quelconque des revendications 12 et 13, dans laquelle un rapport molaire de H₂ à CO est de 1:1 à 10:1, de préférence 2:1.
